# EUROPEAN PATENT APPLICATION

(11) **EP 3 932 334 A1**
(43) Date of publication of application: **05.01.2022**
(21) Application number: 21181735.8
(22) Date of filing: 25.06.2021
(51) Int. Cl.: A61B 17/11, A61B 17/04

(54) **ANASTOMOSIS DEVICE**

(30) Priority: 29.06.2020 US 202063045261 P; 03.08.2020 US 202063060207 P; 16.03.2021 US 202117202623
(71) Applicant: Covidien LP, Mansfield, MA 02048 (US)
(72) Inventor: CHIRUVOLU, Mohan T., 500017 Secunderabad (IN); MANDULA, Rajanikanth, 500040 Hyderabad (IN)
(74) Representative: Maschio & Soames IP Ltd

(57) **Abstract**

An anastomosis device includes a housing that has a first end portion and a second end portion. The first end portion of the housing supports a first suture deployment assembly and the second end portion of the housing supports a second suture deployment assembly. Each of the suture deployment assemblies includes a drive member and a plurality of sutures. Each of the plurality of sutures supports a tissue piercing member. The drive members are engaged with the tissue piercing members and are movable to eject the tissue piercing members and the sutures from the first and second suture deployment assemblies.

## Description

### CROSS-REFERENCE TO RELATED APPLICATIONS

This application claims the benefit of and priority to U.S. Provisional Patent Application No. 63/060,207, filed August 3, 2020 and U.S. Provisional Patent Application No. 63/045,261, filed June 29, 2020, the entire contents of each of which are incorporated by reference herein.

### FIELD

This technology is generally related to an anastomosis device and, more particularly, to a luminal anastomosis device.

### BACKGROUND

Anastomosis devices are commonly used during a variety of surgical procedures to reconnect end portions of resected body vessels. Typically, an anastomosis device includes a circular stapling device having an anvil assembly and a cartridge assembly. The anvil assembly is positioned within one of the end portions of the resected vessel and the cartridge assembly is positioned within the other end portion of the resected vessel and anvil and cartridge assemblies are approximated to clamp the end portions together. The stapling device is actuated to staple the end portions together and core tissue within a lumen defined within the vessel portions.

Known circular stapling devices are not suitable for use in certain surgical procedures in which the body vessel is small such as radical prostatectomy and cystectomy procedures. In such procedures, the cost and skill required to use a circular stapling device is prohibitive. Moreover, the skill required to manually suture the vessel portions in such procedures is extremely high.

A continuing need exists in the art for an anastomosis device that is easy to use and is capable of reconnecting vessel portions having a small diameters.

### SUMMARY

The techniques of this disclosure generally relate to an anastomosis device for joining two vessel portions during an anastomosis procedure. The anastomosis device includes a housing that has a first end portion that supports a first suture deployment assembly and a second end portion that supports a second suture deployment assembly. Each of the suture deployment assemblies includes a drive member and a plurality of sutures. Each of the plurality of sutures supports a tissue piercing member. The drive members are engaged with the tissue piercing members and can be actuated to eject the tissue piercing members and the sutures from the first and second suture deployment assemblies.

One aspect of the disclosure is directed to an anastomosis device that includes a handle assembly, an elongate body, and an end effector. The handle assembly includes an articulation trigger and a firing trigger. The elongate body defines a first longitudinal axis and has a proximal portion and a distal portion. The proximal portion is coupled to the handle assembly. The end effector includes a housing and first and second suture deployment assemblies. The end effector is supported on the distal portion of the elongate body and defines a second longitudinal axis. The housing is pivotably supported on the distal portion of the elongate body and has first and second end portions. The housing is movable from a stowed position in which the first and second longitudinal axes are aligned with each other to a deployed position in which the first and second longitudinal axes are substantially perpendicular to each other. The first suture deployment assembly is supported on the first end portion of the housing and the second suture deployment assembly is supported on the second end portion of the housing. Each of the first and second suture deployment assemblies includes a capsule, a drive member, and a plurality of sutures. Each of the plurality of sutures includes a first end, a second end, and a tissue piercing member secured to the first end of each of the plurality of sutures. Each of the capsules defines a first cavity that receives the drive member and the plurality of sutures. Each of the capsules defines radial openings that communicate with the first cavity. The drive member of each of the first and second deployment assemblies is engaged with the tissue piercing members and is movable from a first position to a second position to advance the tissue piercing members through the radial openings.

Another aspect of the disclosure is directed to an end effector that includes a housing, a first suture deployment assembly, and a second suture deployment assembly. The housing defines a longitudinal axis and has first and second end portions and a central portion including diametrically opposed pivot members. The housing defines a first cavity. The first suture deployment assembly is supported on the first end portion of the housing and the second suture deployment assembly is supported on the second end portion of the housing. Each of the first and second suture deployment assemblies includes a capsule, a drive member, and a plurality of sutures. Each of the plurality of sutures includes a first end, a second end, and a tissue piercing member secured to the first end of each of the sutures. Each of the capsules defines a second cavity that communicates with the first cavity of the housing. The drive member and the plurality of sutures are received within the respective second cavities of the capsules of the first and second suture deployment assemblies. Each of the capsules defines radial openings that communicate with the second cavities. The drive member of each of the first and second suture deployment assemblies is engaged with the tissue piercing members and is movable from a first position to a second position to advance the tissue piercing members through the radial openings.

In aspects of the disclosure, each of the drive members includes a first end and a second end and the second ends of the drive members include a cam member that is engaged with the tissue piercing members such that movement of the drive members from their first positions to their second positions advance the tissue piercing members through the radial openings.

In some aspects of the disclosure, the cam member of each of the drive members is conically shaped.

In certain aspects of the disclosure, the cam members define channels that receive ends of the tissue piercing members.

In aspects of the disclosure, each of the first and second suture deployment assemblies includes a biasing member that urges the respective drive member to its first position.

In some aspects of the disclosure, the elongate body includes an outer tube having a proximal portion and a distal portion, and the distal portion of the outer tube includes an inner surface that defines diametrically opposed longitudinal slots.

In aspects of the disclosure, the housing includes a central portion including pivot members, and the pivot members are received within the diametrically opposed longitudinal slots to facilitate pivotable movement of the housing in relation to the outer tube.

In certain aspects of the disclosure, the diametrically opposed longitudinal slots include closed distal ends.

In aspects of the disclosure, the inner surface of the distal portion of the outer tube includes a transverse slot that communicates with a respective one of the diametrically opposed longitudinal slots, and the housing includes a guide member that is received in each of the diametrically opposed longitudinal slots such that the guide members are movable through the transverse slots as the housing moves from the stowed position to the deployed position.

In some aspects of the disclosure, the second end of each of plurality sutures includes a knot.

In certain aspects of the disclosure, the second ends of the plurality of sutures are coupled together by a suture loop.

In aspects of the disclosure, the anastomosis device includes a firing rod including a proximal portion and a distal portion. The proximal portion of the firing rod is coupled to the firing trigger and the distal portion of the firing rod is engaged with the drive members of the first and second suture deployment assemblies, and the firing rod is movable in response to actuation of the firing trigger from a retracted position to an advanced position to move the drive members from their first positions to their second positions.

In some aspects of the disclosure, the first end of each of the drive members includes a tapered surface, and the tapered surfaces of the drive members are positioned adjacent to each other to define a wedge-shaped recess.

In certain aspects of the disclosure, the distal portion of the firing rod is aligned with the wedge-shaped recess when the firing rod is in its retracted position.

In aspects of the disclosure, the anastomosis device includes a firing cable having a proximal portion and a distal portion. The proximal portion of the firing cable is coupled to the firing trigger and the distal portion of the firing cable is engaged with the drive members of the first and second suture deployment assemblies.

In some aspects of the disclosure, the anastomosis device includes articulation links, and each of the articulation links includes a proximal portion that is coupled to the articulation trigger and a distal portion that is coupled to the housing. The articulation links are movable from retracted positions to an advanced positions to move the housing from the stowed position to the deployed position.

In certain aspects of the disclosure, the channels define slots and the tissue piercing members include ribs that are received within the slots defined within the channels to retain the tissue piercing members within the slots defined within the channels.

In aspects of the disclosure, movement of the drive member from second position back to the first position retracts the tissue piercing members to a position within the capsule.

Other features of the disclosure will be appreciated from the following description.

### BRIEF DESCRIPTION OF DRAWINGS

Various aspects of the disclosure are described herein below with reference to the drawings, wherein:
FIG. 1 is a side perspective view of a surgical anastomosis device according to aspects of the disclosure including an end effector in a non-deployed position;
FIG. 2 is a side perspective view of the surgical anastomosis device shown in FIG. 1;
FIG. 3 is a side perspective cross-sectional view of the surgical anastomosis device shown in FIG. 1 in the non-deployed position;
FIG. 4 is an enlarged view of the indicated area of detail shown in FIG. 1;
FIG. 5 is an enlarged view of the indicated area of detail shown in FIG. 2;
FIG. 6 is a side perspective exploded view of an end effector of the surgical anastomosis device shown in FIG. 1;
FIG. 7 is a cross-sectional view taken along section line 7-7 of FIG. 4;
FIG. 8 is a cross-sectional view taken along section line 8-8 of FIG. 7;
FIG. 9 is a side perspective view of an outer shaft of the surgical anastomosis device shown in FIG. 1;
FIG. 10 is a cross-sectional view taken along section line 10-10 of FIG. 7;
FIG. 11 is a side perspective view of the surgical anastomosis device shown in FIG. 1 with an elongate body of the surgical anastomosis device inserted through a cannula into a body cavity of a patient;
FIG. 12 is a side partial cross-sectional view of the surgical anastomosis device shown in FIG. 1 as the end effector is moved to the deployed position with the end effector positioned within spaced vessel portions;
FIG. 13 is a cross-sectional view taken through a distal portion of the surgical anastomosis device with the end effector in the deployed position;
FIG. 14 is a side cutaway view of a handle assembly of the surgical anastomosis device shown in FIG. 1 in an actuated position;
FIG. 15 is a side partial cross-sectional view of the surgical anastomosis device shown in FIG. 1 as the end effector is moved to the fired position with the end effector positioned within the spaced vessel portions;
FIG. 16 is a side perspective view of the spaced vessel portions after sutures of the surgical anastomosis device shown in FIG. 1 are deployed in the vessel portions;
FIG. 17 is a side perspective view of the spaced vessel portions after the vessel portions are joined together;
FIG. 18 is a side perspective partial cross-sectional view with a portion of the handle assembly removed of an alternative version of the surgical anastomosis device shown in FIG. 1;
FIG. 19 is an enlarged view of the indicated area of detail shown in FIG. 18:
FIG. 20 is a cross-sectional view taken along section line 20-20 of FIG. 19;
FIG. 21 is a side perspective exploded view of the end effector of the surgical anastomosis device shown in FIG. 18;
FIG. 22 is a side perspective view of an outer shaft of the surgical anastomosis device shown in FIG. 18;
FIG. 23 is a side partial cross-sectional view of the surgical anastomosis device shown in FIG. 18 as the end effector is moved to the fired position with the end effector positioned within the spaced vessel portions;
FIG. 24 is a side perspective view of the spaced vessel portions after sutures of the surgical anastomosis device shown in FIG. 18 are deployed in the vessel portions;
FIG. 25 is a side perspective view of the spaced vessel portions after the vessel portions are joined together;
FIG. 26 is a side perspective view of another version of a drive member and tissue piercing members of a suture deployment assembly of the anastomosis device shown in FIG. 1;
FIG. 27 is an enlarged view of one of the tissue piercing members shown in FIG. 26;
FIG. 28 is an enlarged view of the indicated area of detail shown in FIG. 26;
FIG. 29 is a side perspective view of a portion of one of the drive member members shown in FIG. 26 with the tissue piercing members in a non-deployed position;
FIG. 30 is a cross-sectional view taken through one of the suture deployment assemblies of the anastomosis device shown in FIG. 1 in a pre-fired non-deployed position;
FIG. 31 is a cross-sectional view taken through the suture deployment assemblies shown in FIG. 30 in the deployed position;
FIG. 32 a cross-sectional view taken through the suture deployment assembly of the anastomosis device shown in FIG. 1 in a post fired retracted position;
FIG. 33 is a side perspective view of the spaced vessel portions after sutures of the anastomosis device shown in FIG. 1 are deployed in the vessel portions;
FIG. 34 is a side perspective view of another version of a drive member and tissue piercing members of a suture deployment assembly of the anastomosis device shown in FIG. 1 with the tissue piercing members spaced from the drive member; and
FIG. 35 is a side perspective view of the drive member and tissue piercing members shown in FIG. 34 with the tissue piercing members spaced supported on the drive member in the non-deployed position.

### DETAILED DESCRIPTION

The disclosed surgical anastomosis device will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through small diameter incision or cannula. Further, the term "clinician" is used generally to refer to medical personnel including doctors, nurses, and support personnel.

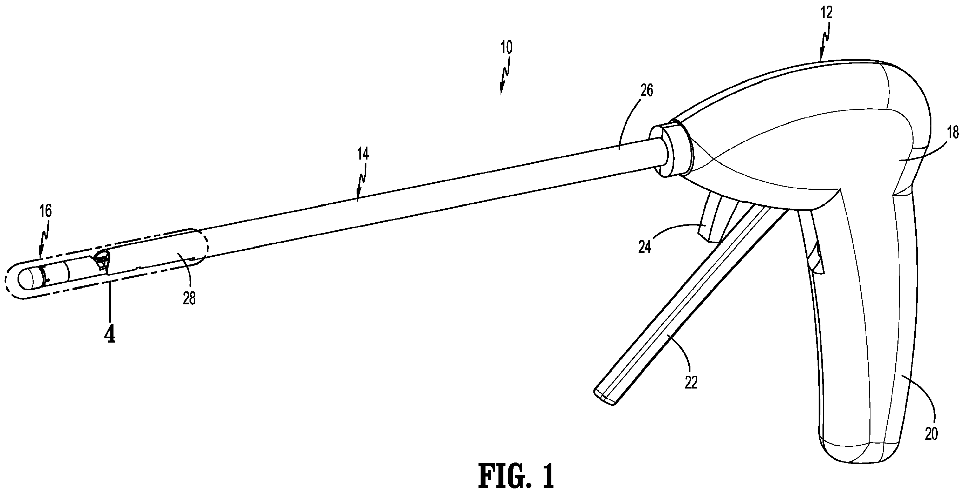

FIG. 1 illustrates the disclosed surgical anastomosis device shown generally as anastomosis device 10. The anastomosis device 10 includes a handle assembly 12, an elongate body 14, and an end effector 16. The handle assembly 12 includes a body portion 18 that defines a stationary handle or grip 20, an articulation trigger 22, and a firing trigger 24. Although the handle assembly 12 is illustrated as a pistol-type grip, it is envisioned that a variety of grip configurations can be used with the anastomosis device 10. The elongate body 14 includes a proximal portion 26 that is coupled to the handle assembly 12 and a distal portion 28 that supports the end effector 16.

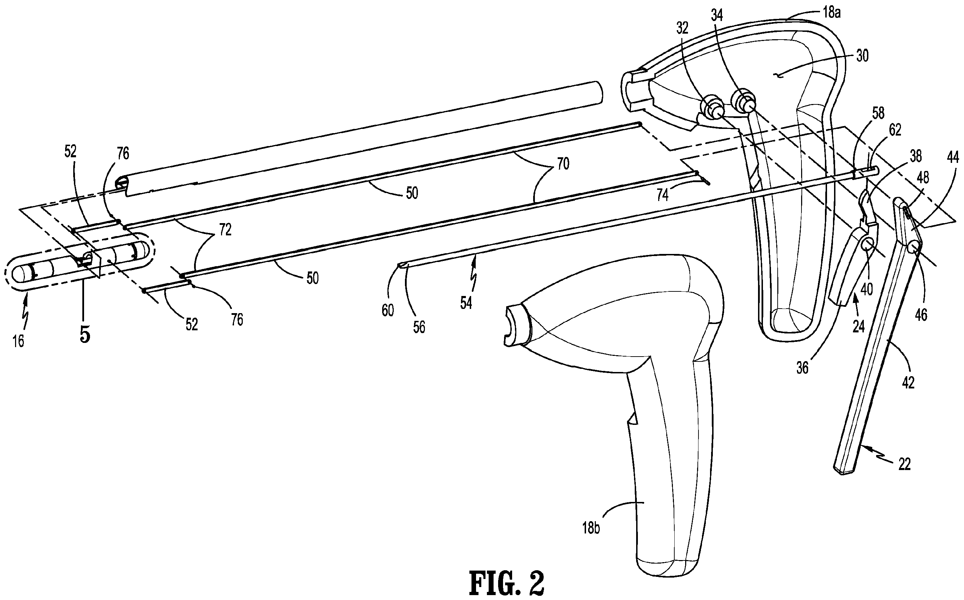

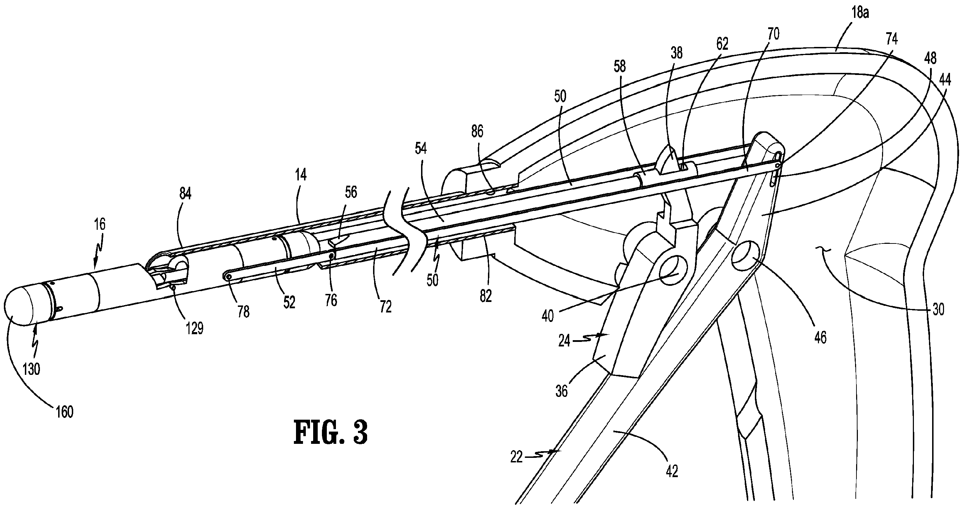

FIGS. 2 and 3 illustrate an exploded view of the anastomosis device 10. The body portion 18 of the handle assembly 12 (FIG. 1) is formed from molded half-sections 18a and 18b. The molded half-sections 18a and 18b are secured together using known securements techniques, e.g., ultrasonic welding, and define a cavity 30 that receives internal components of the handle assembly 12. At least one of the molded half-sections 18a and 18b includes first and second pivot members 32 and 34. In aspects of the disclosure, the first and second pivot members 32 and 34 are integrally or monolithically formed with the respective molded half-section 18a and/or 18b. Alternately, the pivot members 32 and 34 can be formed separately from and secured to the respective body portion 18a and/or 18b.

The firing trigger 24 is pivotally mounted on the first pivot member 32 and includes a finger engagement portion 36 and a link portion 38. The firing trigger 24 defines a through bore 40 that is positioned between the finger engagement portion 36 and the link portion 38 and receives the first pivot member 32. The engagement portion 36 of the firing trigger 24 is pivotable towards the stationary handle 20 to move the link portion 38 of the firing trigger 24 in the distal direction.

The articulation trigger 22 is pivotally mounted on the second pivot member 34 and includes a finger engagement portion 42 and a link portion 44. The articulation trigger 24 defines a through bore 46 that is positioned between the finger engagement portion 42 and the link portion 44 and receives the second pivot member 34. The engagement portion 42 of the articulation trigger 22 is pivotable towards the stationary handle 20 to move the link portion 44 of the articulation trigger 22 in the distal direction. The link portion 44 of the articulation trigger 22 defines an elongated slot 48.

The anastomosis device 10 includes first articulation links 50, second articulation links 52, and a firing rod 54. The firing rod 54 is positioned between the first articulation links 50 and includes a distal portion 56 and a proximal portion 58. The distal portion 56 of the firing rod 54 includes a tapered distal end 60. The proximal portion 58 of the firing rod 54 defines a slot 62 that receives the link portion 38 of the firing trigger 24 such that that actuation of the firing trigger 24, i.e., movement of the finger engagement portion 36 of the firing trigger 24 towards the stationary handle 20 of the handle assembly 12, moves the firing rod 54 of the anastomosis device 10 from a retracted position towards an advanced position.

Each of the first articulation links 50 has proximal portion 70 and a distal portion 72. The proximal portions 70 of the first articulation links 50 are coupled to each other and to the link portion 44 of the articulation trigger 22 by a pin 74. The pin 74 extends through the elongated slot 48 formed in the link portion 44 of the articulation trigger 22. The elongated slot 48 allows the link portion 44 of the articulation trigger 22 to translate pivotable movement of the articulation trigger 22 into longitudinal movement of the first articulation links 50 when the articulation trigger 42 is actuated, i.e., when the engagement portion 42 of the articulation trigger 22 is pivoted towards the stationary handle 18 of the handle assembly 12.

Each of the second articulation links 52 includes a proximal portion and a distal portion. The proximal portion of each of the second articulation links 52 is coupled to the distal portion 72 of a respective one of the first articulation links 50 by a pivot member 76. The distal portion of each of the second articulation links 52 is pivotably coupled to the end effector 16 by a pivot member 78 (FIG. 3).

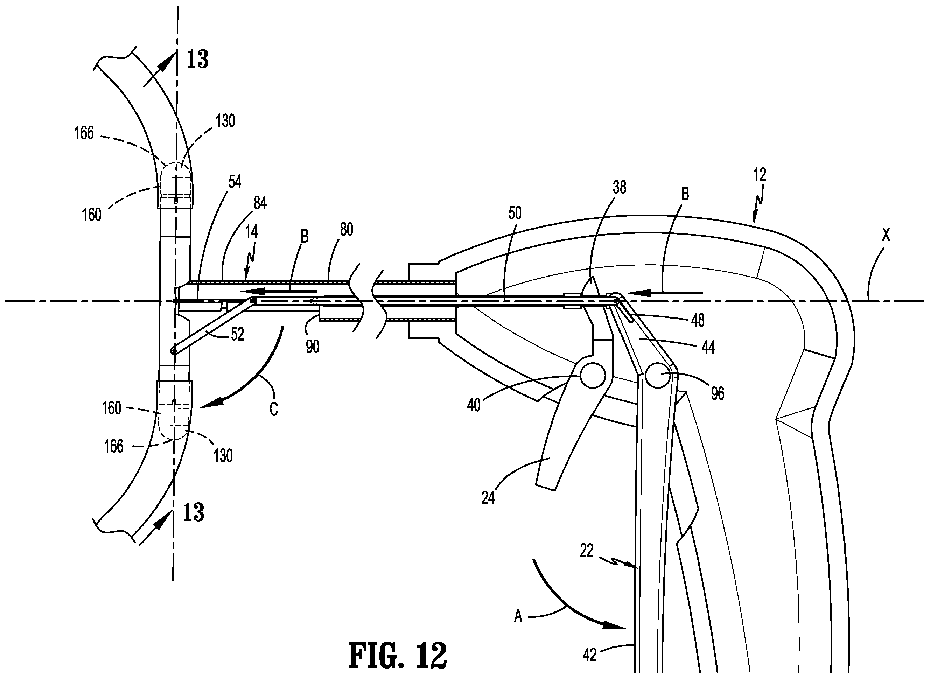

The articulation trigger 22 can be actuated to advance the first articulation links 50 from retracted positions to advanced positions. When the first articulation links 50 are moved to their advanced positions, the second articulation links 52 are also moved from retracted positions to advanced positions. As described above, the second articulation links 52 are pivotably coupled to the distal portions 72 of the first articulation links 50 and to the end effector 16. This arrangement allows the end effector 16 to articulate in relation to the elongate body 14 from a stowed position (FIG. 1) to a deployed position (FIG. 12) as described in further detail below.

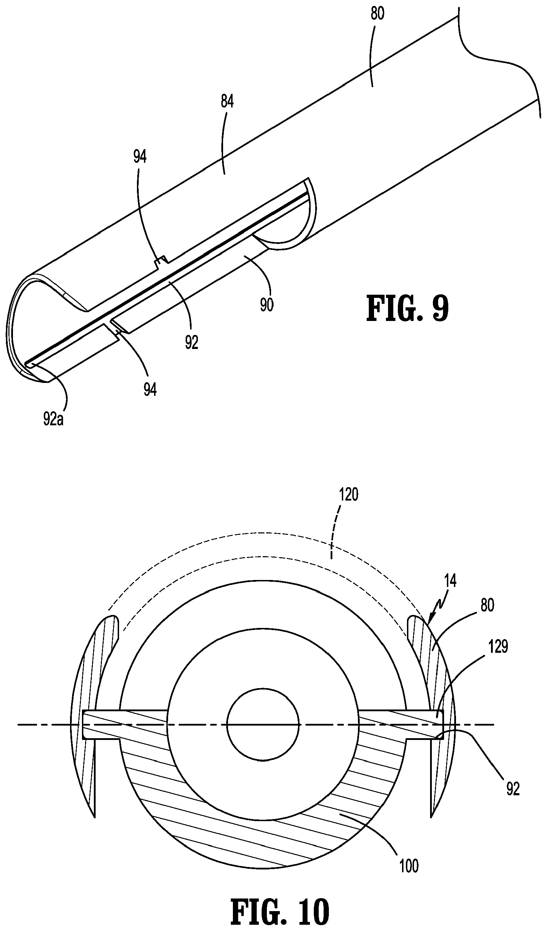

The elongate body 14 includes an outer tube 80 that has a proximal portion 82 and a distal portion 84. The proximal portion 82 of the outer tube 82 of the elongate body 14 is fixedly secured within an opening 86 (FIG. 3) in the body portion 18 of the handle assembly 12. The distal portion 84 of the outer tube 80 of the elongate body 14 receives the end effector 16 when the end effector 16 is in its stowed position and includes an elongated cutout 90 (FIG. 9) in the distal end of the distal portion 84 that allows the end effector 16 to pivot in relation to the outer tube 80 when the end effector 16 is moved from its stowed position to its deployed position.

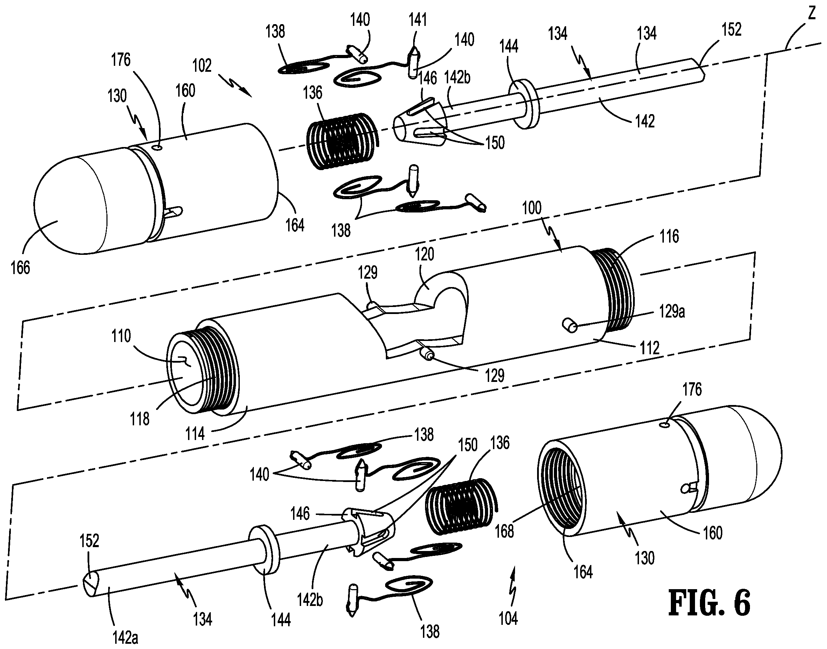

The distal portion 84 of the outer tube 80 defines diametrically opposed longitudinal slots 92 (FIG. 9) that receive pivot members 129 and guide members 129a (FIG. 6) of the end effector 16 as described in detail below to guide and pivotably support the end effector 16 as the end effector 16 moves from its stowed position to its deployed position. The opposed longitudinal slots 92 have a closed distal end 92a (FIG. 9) to prevent distal movement of the pivot members 129 within the slots 92 beyond the closed distal end 92a. The distal portion 84 of the outer tube 80 also includes two transverse slots 94 that allow pivot members 129 and guide members 129a of the end effector 16 to be received within the longitudinal slots 92 during assembly of the anastomosis device 10 (FIG. 1). The transverse slots 94 also allow the guide members 129a to exit the longitudinal slots 92 when the end effector 16 is pivoted to the deployed position.

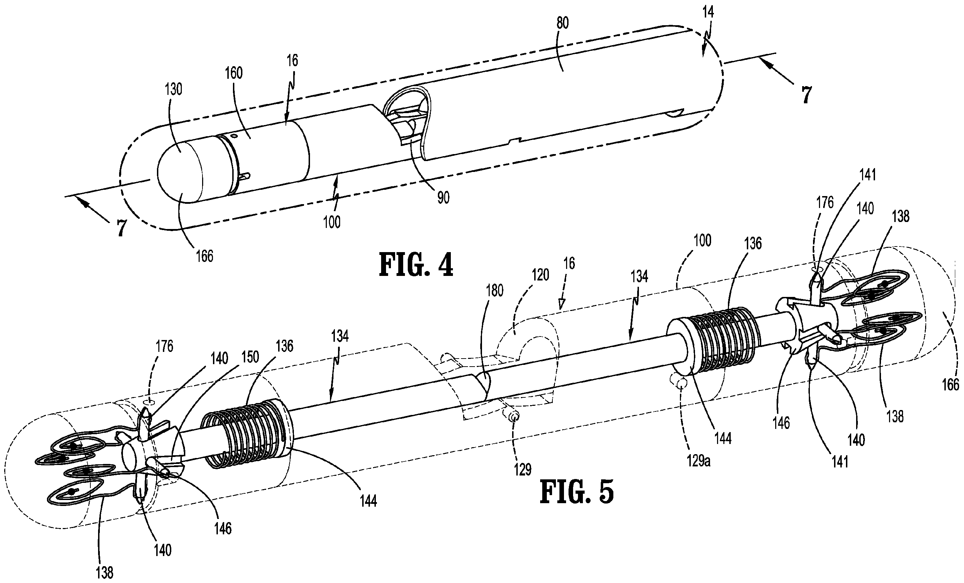

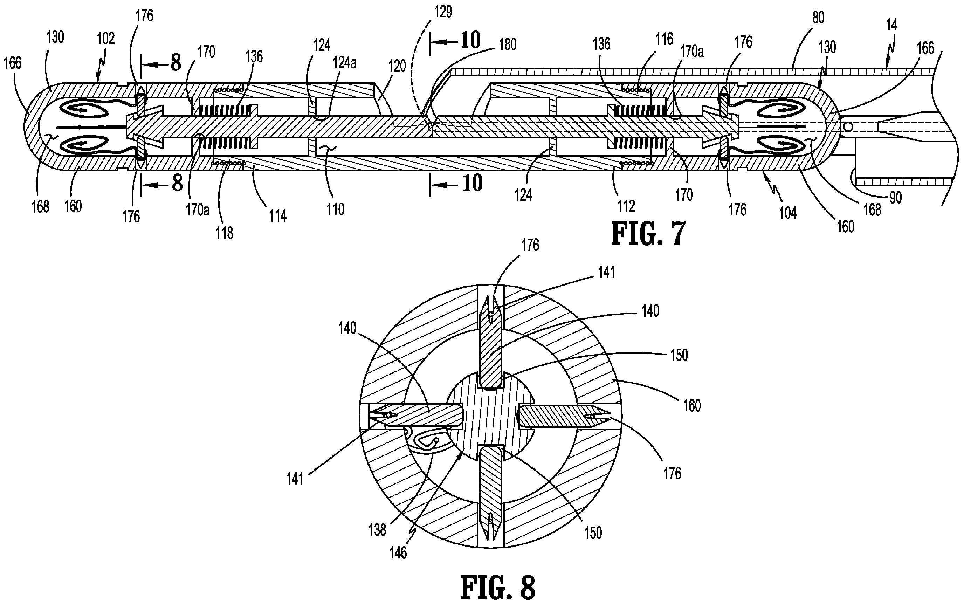

FIGS. 4-10 illustrate the end effector 16 of the anastomosis device 10 which includes a housing 100, and first and second suture deployment assemblies 102 and 104 supported on each end of the housing 100. The suture deployment assemblies 102 and 104 are identical to each other. As such, only suture deployment assembly 102 will be described in detail in this disclosure.

The housing 100 of the end effector 16 defines a cavity 110 and includes an open proximal end 112 and an open distal end 114. The open proximal and distal ends 112 and 114 communicate with the cavity 110. The open proximal end 112 includes an outer threaded portion 116 and the open distal end 114 includes an outer threaded portion 118. The threaded portions 116 and 118 engage the first and second suture deployment assemblies 102 and 104 as described below. The housing 100 defines a centrally located opening 120 that also communicates with the cavity 110 and an annular flange 124 within the cavity 110 on each side of the opening 120. Each of the annular flanges 124 defines a cylindrical bore 124a. The housing 100 also supports centrally located pivot members 129 and guide members 129a that are positioned on opposite sides of the opening 120 and extend radially outward from the housing 100.The pivot members 129 and the guide members 129a are receive within the longitudinal slots 92 of the outer tube 80 on the elongate body 14.

The suture deployment assemblies 102 and 104 each include a capsule 130 having an open threaded end 132, a drive member 134, a biasing member 136, and a plurality of sutures 138. Although four sutures 138 are shown in each of the suture deployment assemblies 102 and 104, it is envisioned that each of the suture deployment assemblies can include two or more sutures 138. Each of the sutures 138 includes a dart or tissue piercing member 140 secured to one end of the suture 138. Each of the tissue piercing members 140 includes a tapered tip 141 for piercing tissue. The capsules 130 are secured to the threaded ends of the housing 100 of the end effector 16.

The drive member 134 of each of the suture deployment assemblies 102 and 104 includes a cylindrical shaft 142 having a first end 142a and a second end 142b. The cylindrical shaft 142 defines a longitudinal axis "Z" (FIG. 6) and includes a centrally positioned annular flange 144 and a cam member 146. The cam members 146 are substantially conical in shape and define a plurality of longitudinally extending channels 150. Each of the longitudinally extending channels 150 receives the end of a respective one of the tissue piercing members 140 opposite to the tapered tip 141.

The drive member 134 of each of the suture deployment assemblies 102 and 104 is supported within the housing 100 of the respective suture deployment assembly 102 or 104 and extends through the cylindrical bore 124a of the annular flange 124 of the housing 100. The first end 142a of the drive member 134 includes a tapered surface 152.

Each of the capsules 130 includes a capsule housing 160 that defines a cavity 168 and includes a cylindrical body portion 162 having a threaded open end 164 and a closed blunt end 166. In aspects of the disclosure, the blunt end 166 has a spherical configuration although other configurations are envisioned. The capsule housing 160 includes an annular flange 170 that defines a through bore 170a that receives the cylindrical shaft 142 of the shaft drive member 134. The threaded open end 164 of each of the capsule housings 160 is secured to one of the threaded portions 116 and 118 of the housing 100 of the end effector 16 such that the cavity 168 of the of the capsule housing 160 communicates with the cavity 110 of the housing 100. When the drive members 134 are supported within the end effector 16, the annular flanges 144 of the drive members 134 are spaced from the annular flanges 170 of the capsule housings 160.

The capsule housings 160 define radially extending openings 176. Each of the radially extending openings 176 in the capsule housings 160 receives one of the tissue piercing members 140. The cam member 146 of each of the drive members 134 is positioned within the respective capsule housing 160 and is aligned with the openings 176 such that the ends of the tissue piercing members 140 opposite the tapered ends 141 are received within the respective channels 150 of the respective cam member 146 and the tapered ends 141 of the tissue piercing members 140 are received within the openings 176 in the respective capsule housing 160. As described above, the cam member 146 of each of the drive members 134 is conical in shape. The drive members 134 are movable from a first position in which the tissue piercing members 140 are received within a first small diameter end of the channel 150 of the cam member 146 to a second position in which the tissue piercing members 140 are received within a second large diameter end of the channel 150 of the cam member 146. As the drive members 134 are moved from their first positions to their second positions, the piercing members 140 are forced outwardly through the openings 176 in the capsule housings 160.

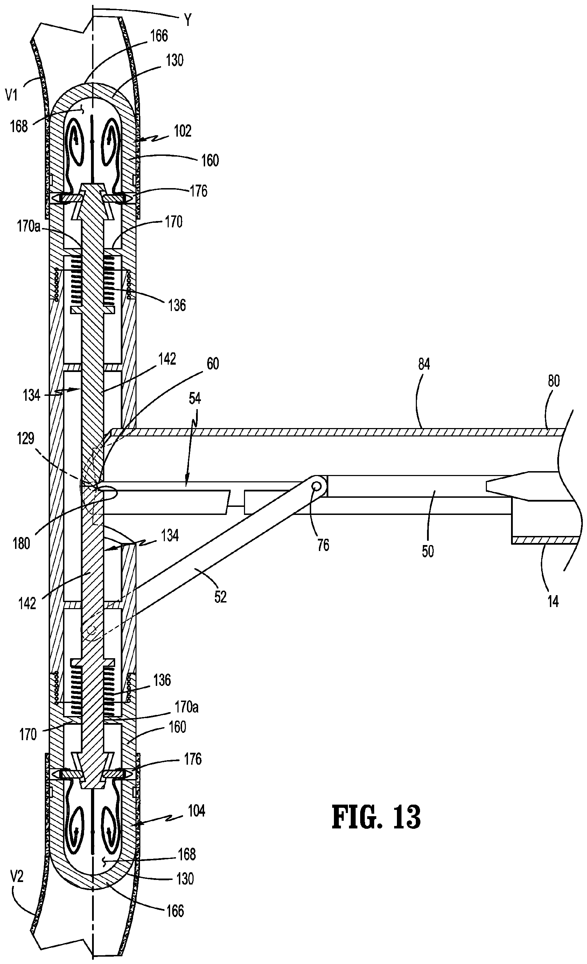

Each of the biasing members 136 of the suture deployment assemblies 102 and 104 is positioned within the cavities 110 and 168 of the housing 100 and the capsule housing 160, respectively, about the cylindrical shaft 142 of the drive members 134 between the flange 144 of the drive member 134 and the flange 170 of the capsule housing 160 to urge the drive members 134 towards their first positions. In aspects of the disclosure, the biasing members 136 are coil springs. Alternately, other types of biasing members could be employed. When the drive members 134 are in their first positions, the first ends 142a of the drive members 134 are engaged with each other such that the tapered surfaces 152 of the drive members 134 define a wedge-shaped recess 180 (FIG. 13) that is aligned with the tapered distal end 60 of the firing rod 54.

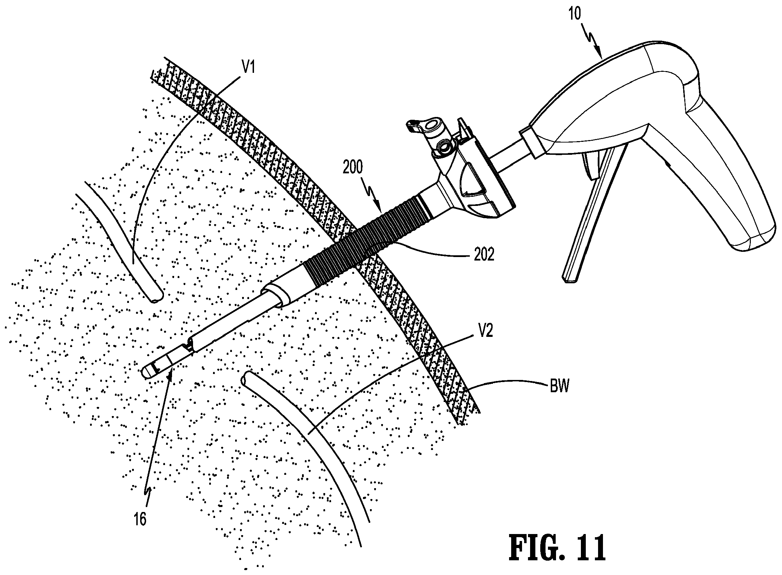

FIG. 11 illustrates the anastomosis device 10 with the end effector 16 inserted through a cannula 200 that is inserted through an incision 202 in a body wall "BW" of a patient. In aspects of the disclosure, the diameter of the elongate body 14 of the anastomosis device 10 is between about 8 mm and about 10 mm. Alternately, the elongate body 14 of the anastomosis device 10 can have other diameters, e.g., 5mm, 12mm, etc. In use, the anastomosis device 10 is inserted through the cannula 200 and the end effector 16 is positioned adjacent to vessel portions "VI" and V2" that need to be joined.

FIGS. 12 and 13 illustrate the end effector 16 as the end effector 16 is moved from the stowed position to the deployed position. In order to deploy the end effector 16, the articulation trigger 22 is actuated (pivoted in the direction of arrow "A" in FIG. 12) to advance the first articulation links 50 in the direction of arrows "B" from their retracted positions to their advanced positions. As the first articulation links 50 move longitudinally within the outer tube 80 of the elongate body 14 of the anastomosis device 10, the second articulation links 52 are advanced distally to advance the end effector 16 from the distal portion 84 of the outer tube 80 of the elongate body 14. As the end effector 16 is advanced within the outer tube 80, the pivot members 129 (FIG. 5) and the guide members 129a move longitudinally within the longitudinal slots 92 formed in the distal portion 84 of the outer tube 80. When the pivot members 129 reach the closed ends 92a (FIG. 9) of the slots 92 and the guide members 129a become aligned with the transverse slots 94, further advancement of the first and second articulation links 50 and 52 causes the end effector 16 to rotate about the pivot members 129 in the direction indicated by arrow "C" in FIG. 12 to the deployed position. In the deployed position, the end effector defines an axis "Y" that is substantially perpendicular to the longitudinal axis "X" of the elongate body 14 and the tapered distal end 60 of the firing rod 54 is positioned within the wedge-shaped recess 180 defined between the tapered surfaces 152 of the drive member 134.

When the end effector 16 is positioned in the deployed position, the suture deployment assemblies 102 and 104 are inserted into the respective vessel portions "VI" and V2" (FIG. 13). The suture deployment assemblies 102 and 104 are positioned within the vessel portions "VI" and "V2" to a depth to cover the radially extending openings 176 in the capsule housings 160. The blunt ends 166 of the capsule housings 160 of the capsules 130 allow the suture deployment assemblies 102 and 104 to enter the vessel portions "VI" and "V2" without damaging the vessel portions "VI" and "V2".

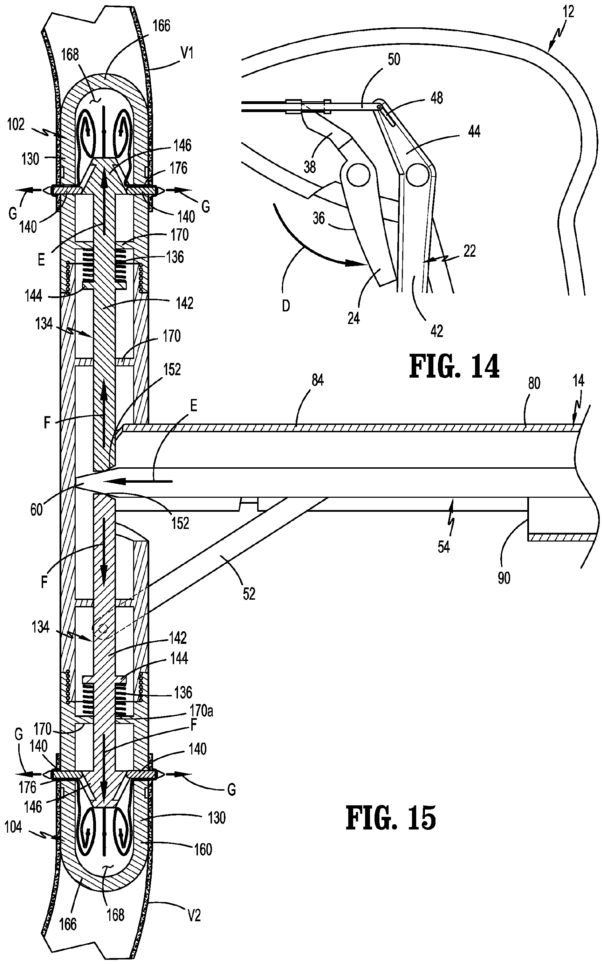

FIGS. 14 and 15 illustrate the anastomosis device 10 as the anastomosis device 10 is fired to deploy the tissue piercing members 140 from the capsules 130. In order to fire the tissue piercing members 140 from the capsules 130 of the suture deployment assemblies 102 and 104, the firing trigger 24 is actuated (pivoted towards the stationary handle 18 in the direction of arrow "D" in FIG. 14) to move the firing rod 54 from its retracted position to its advanced position in the direction of arrow "E" in FIG. 15. When the firing rod 54 moves to its advanced position, the tapered distal end 60 of the firing rod 54 is advanced into the wedged-shaped recess 180 (FIG. 13) defined between the tapered surfaces 152 of the drive members 134. As the tapered distal end 60 of the firing rod 54 moves through the recess 180, the tapered distal end 60 of the firing rod 54 engages the tapered surfaces 152 of the drive members 134 to move the drive members 134 into the capsules 130 and move the cam members 146 in the direction of arrows "F" in FIG. 15 in relation to the tissue piercing members 140. As the cam members 146 move in relation to the tissue piercing members 140, the ends of the tissue piercing members 140 move within the channels 150 and are forced radially outwardly from the openings 176 in the direction of arrows "G" through the vessel portions "VI" and "V2".

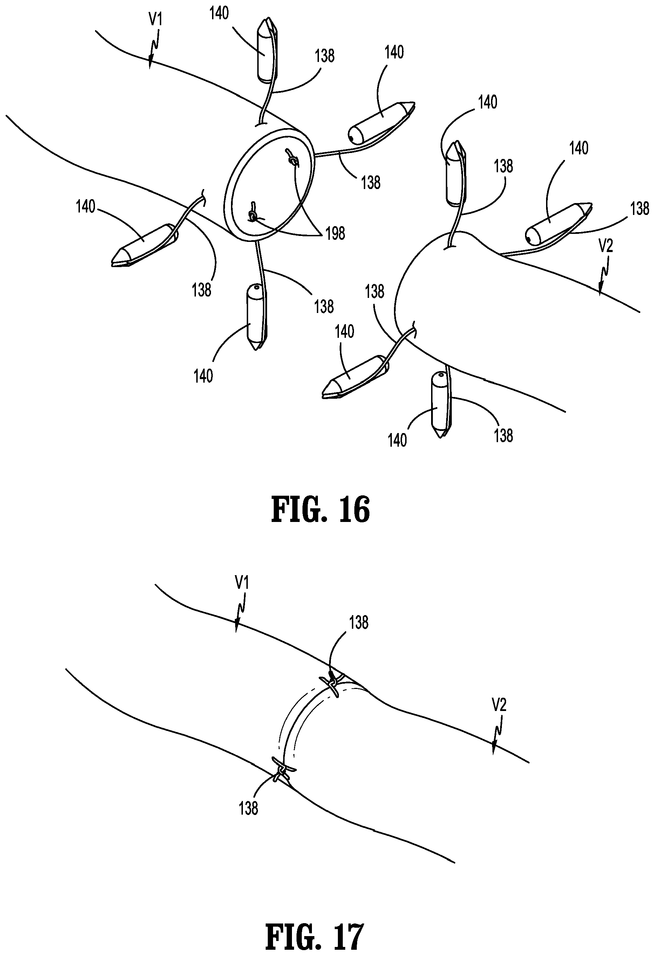

FIG. 16 illustrates the vessel portions "VI" and "V2" with the sutures 138 secured to the tissue piercing members 140. As illustrated, the ends of the sutures 138 opposite to the tissue piercing members 140 include knots 198 that prevent the sutures 138 from passing through the openings in the vessel portions "VI" and "V2" formed by the tissue piercing members 140. As shown in FIG. 17, after the tissue piercing members 140 are passed through the vessel portions "VI" and "V2", the tissue piercing members 140 are removed from the sutures 138 and the sutures 138 from the vessel portion "VI" are secured or tied to the sutures 138 from the vessel portion "V2" to secure the vessel portions "VI" and "V2" together.

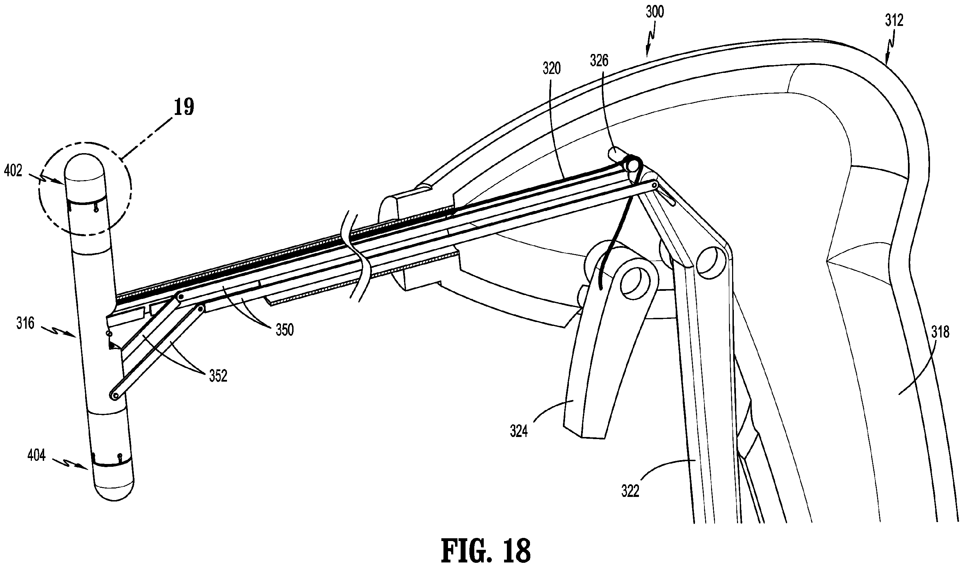

FIGS. 18-23 illustrate an alternate version of the disclosed anastomosis device shown generally as anastomosis device 300. The anastomosis device 300 is substantially similar to the anastomosis device 10 except that the firing mechanism and the suture have been modified. As such, only the differences between the anastomosis device 10 and the anastomosis device 300 will be described in detail herein.

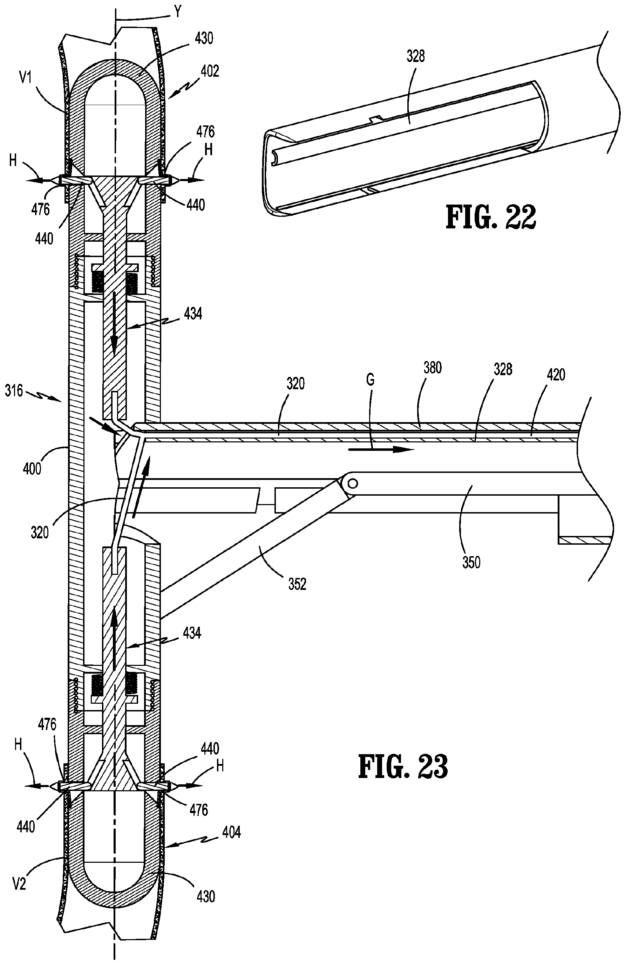

The anastomosis device 300 includes a handle assembly 312 having an articulation trigger 322 and a firing trigger 324. The articulation trigger 322 is coupled to articulation links 350 and 352 which are coupled to the end effector 316 as described above regarding the anastomosis device 10 and will not be described in further detail herein. The firing trigger 324 is pivotably supported on a body portion 318 of the handle assembly 12. A firing cable 320 has a first end that is coupled to the firing trigger 324 and a second end that is coupled to the first and second suture deployment assemblies 402 and 404. The firing cable 320 is directed about a post 326 that is positioned on the body portion 318 of the anastomosis device 300 and extends through a channel 328 (FIG. 22) formed on an inner wall of the outer tube 380 of the elongate body 314 of the anastomosis device 300 such that actuation of the firing trigger 324 moves the firing cable 320 proximally within the elongate body 314 of the anastomosis device 300.

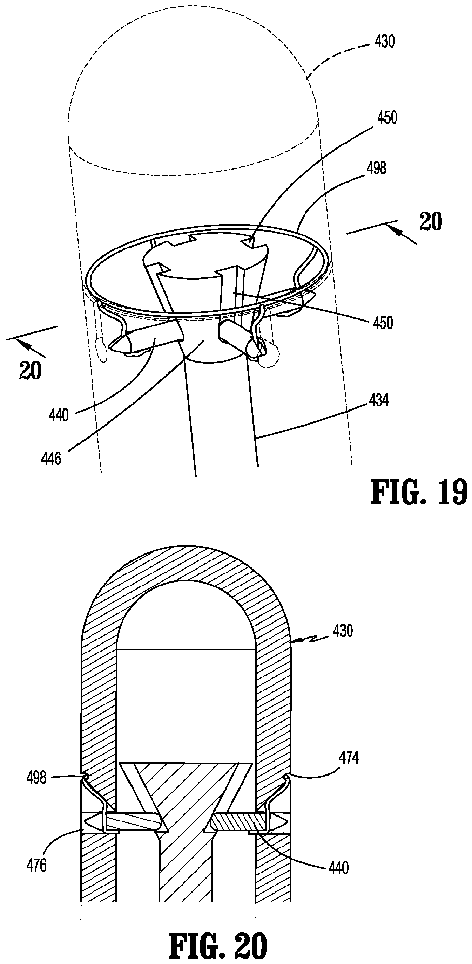

FIGS. 19-21 illustrate the differences between the anastomosis device 300 and the anastomosis device 10. In the anastomosis device 300, the end effector 316 includes a housing 400, and first and second suture deployment assemblies 402 and 404 supported on each end of the housing 400. The housing 400 is substantially the same as the housing 100 (FIG. 6) and will not be described in further detail herein. The suture deployment assemblies 402 and 404 each include a capsule 430 having an open threaded end 432, a drive member 434, a biasing member 436, and a plurality of sutures 438. Although four sutures 438 are shown in each of the suture deployment assemblies 402 and 404, it is envisioned that each of the suture deployment assemblies can include two or more sutures 438. Each of the sutures 438 includes a dart or tissue piercing member 440 secured to one end of the suture 438. Each of the tissue piercing members 440 includes a tapered tip 441 for piercing tissue.

The suture deployment assemblies 402 and 404 operate in a similar fashion to suture deployment assemblies 102 and 104 (FIG. 6) except that the direction of movement of the drive members 434 is changed. In the suture deployment assemblies 102 and 104, the drive members are pushed or cammed from their first positions to their second positions by the firing rod 54 (FIG. 6). In contrast, in the suture deployment assemblies 402 and 404, the drive members 434 are pulled by the firing cable 420 from their first positions to their second positions. In order to account for this change in direction of movement, the configuration of the cam members 446 on the end of the drive members 434 is reversed. In all other respects, the cam members 446 of the drive members 434 are identical to the cam members 146 of the drive members 134. More specifically, the cam members 446 of the drive members 434 are conically shaped and define channels 450 that receive ends of the tissue piercing members 440.

The sutures 438 are substantially similar to the sutures 138 (FIG. 6) except that the knot 198 (FIG. 16) has been replaced by a suture loop 498. More specifically, each of the sutures 438 is connected to each of the other sutures 438 by a suture loop 498. In some aspects of the disclosure, the suture loop 498 is received within an annular groove 474 formed about the capsule 430 adjacent the radial openings 476.

FIG. 23 illustrates the end effector 316 as the sutures 438 are fired from the capsules 430. When the firing trigger 324 is actuated, the firing cable 420 is pulled proximally within the elongate body 314 of the anastomosis device 300 in the direction of arrow "G". As the firing cable is pulled proximally, the drive members 434 of the suture deployment assemblies 402 and 404 are pulled towards each other along the longitudinal axis "Y" of the end effector 16 to move the cam members 446 in relation to the tissue piercing members 440. As the cam members 446 move in relation to the tissue piercing members 440, due to the configuration of the cam members 446, the tissue piercing members 440 are cammed outwardly in the directions of arrows "H" through the openings 476 through the vessel portions "VI" and "V2".

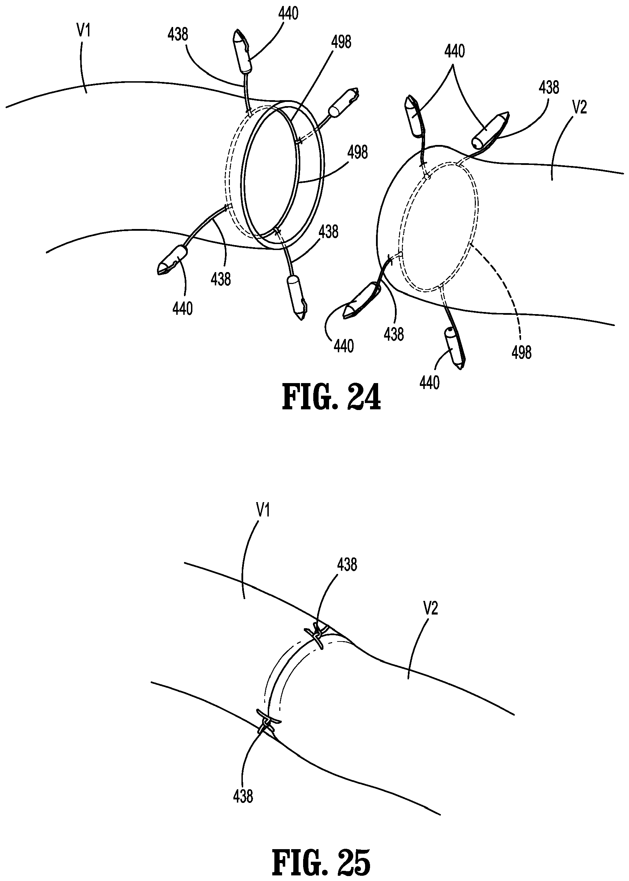

FIG. 24 illustrates the vessel portions "VI" and "V2" with the sutures 438 secured to the tissue piercing members 440. As illustrated, the ends of the sutures 438 opposite to the tissue piercing members 440 are coupled to the suture loop 498 that prevent the sutures 438 from passing through the openings in the vessel portions "VI" and "V2" formed by the tissue piercing members 440. As shown in FIG. 25, after the tissue piercing members 440 are passed through the vessel portions "VI" and "V2", the tissue piercing members 440 are removed from the sutures 438 and the sutures 438 from the vessel portion "VI" are secured to the sutures 438 from the vessel portion "V2" to secure the vessel portions "VI" and "V2" together.

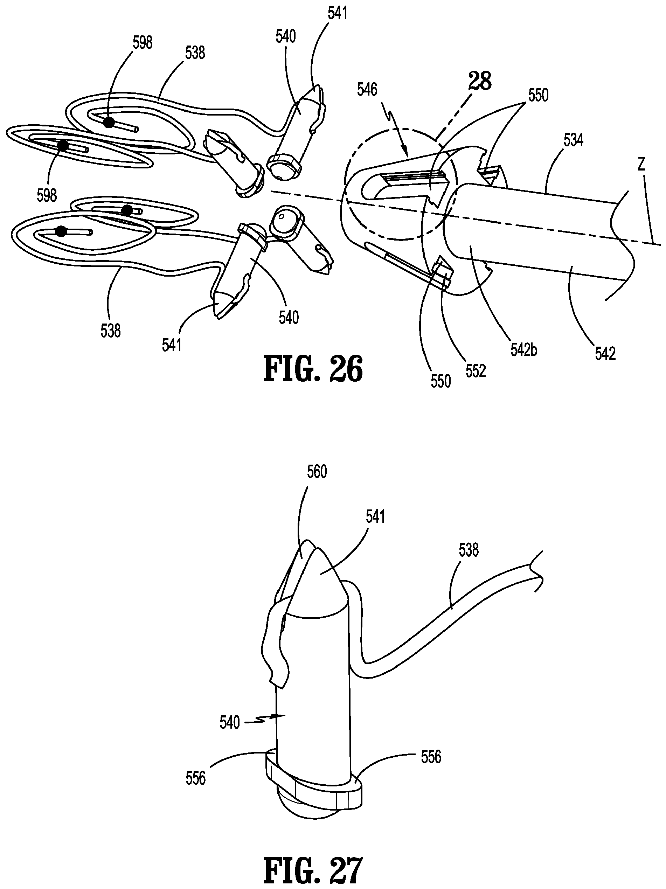

FIGS. 26-35 illustrate another version of a drive member and tissue piercing members of a suture deployment assembly of the anastomosis device shown in FIG. 1. Each of the tissue piercing members is shown generally as 540 and includes a tapered tip 541 for piercing tissue. The drive members (only one is shown) shown generally as drive member 534 includes a cylindrical shaft 542 having a first end (not shown) and a second end 542b. The cylindrical shaft 542 defines a longitudinal axis "Z" (FIG. 26) and supports a cam member 546 supported on the second end 542b of the cylindrical shaft 542. The cam member 546 is substantially conical in shape and defines a plurality of longitudinally extending channels 550. The large diameter end of the cam member 546 is coupled to or formed integrally with the drive member 534. Each of the longitudinally extending channels 550 receives an end of a respective one of the tissue piercing members 540 opposite to the tapered tip 541.

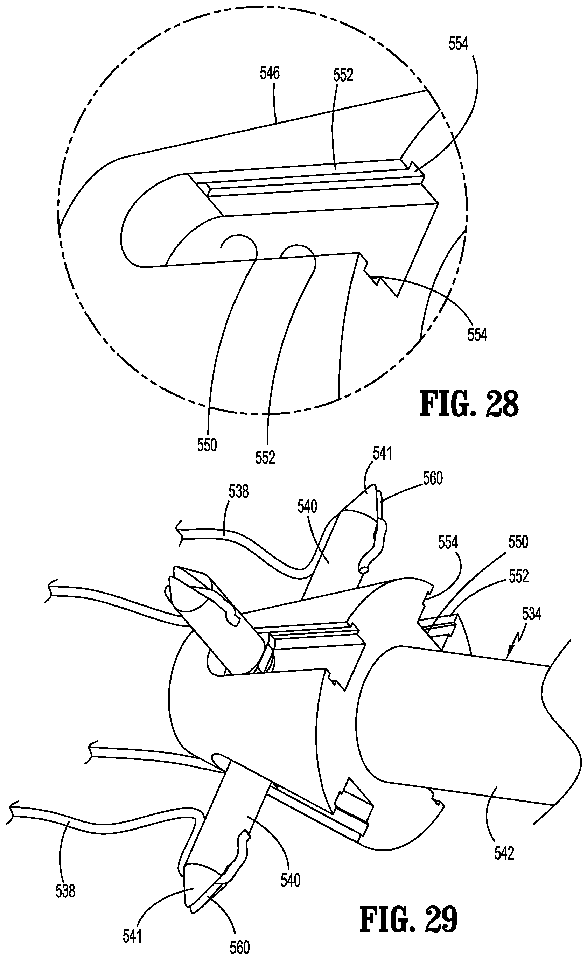

The cam member 546 and tissue piercing members 540 are substantially similar to the drive members 134 (FIG. 5) and tissue piercing members 140 (FIG. 6) described above except that the tissue piercing members 540 are retained within the longitudinally extending channels 550 of the cam members 546. More particularly, each of the longitudinal channels 550 are defined by spaced side walls 552 (FIG. 28) that define elongated slots 554 (FIG. 28) and each of the tissue piercing members 540 includes a rib 556 (FIG. 27) that is received within each of the elongated slots 554. Receipt of the ribs 556 within the slots 554 retains the tissue piercing members 540 within the longitudinal channels 550 but allows the tissue piercing members 540 to move within the longitudinal channels 550 along the longitudinal axis Z (FIG. 26) of the drive member 534 between non-deployed and deployed positions. In aspects of the disclosure, the ribs 556 can be formed by a single annular rib that extends about the tissue piercing member 540.

Each of the tissue piercing members 540 includes a slot 560 (FIG. 27) that is formed in the tapered tip 541. The slot 560 in each of the tissue piercing members 540 receives a first end of a suture 538. In aspects of the disclosure, the sutures 538 are frictionally retained within the slots 560. Alternately, other securement devices or means can be provided to releasably retain the sutures 538 within the slots 560 of the tissue piercing members 540. The sutures 538 each include a second end that includes a knot 598 (FIG. 26).

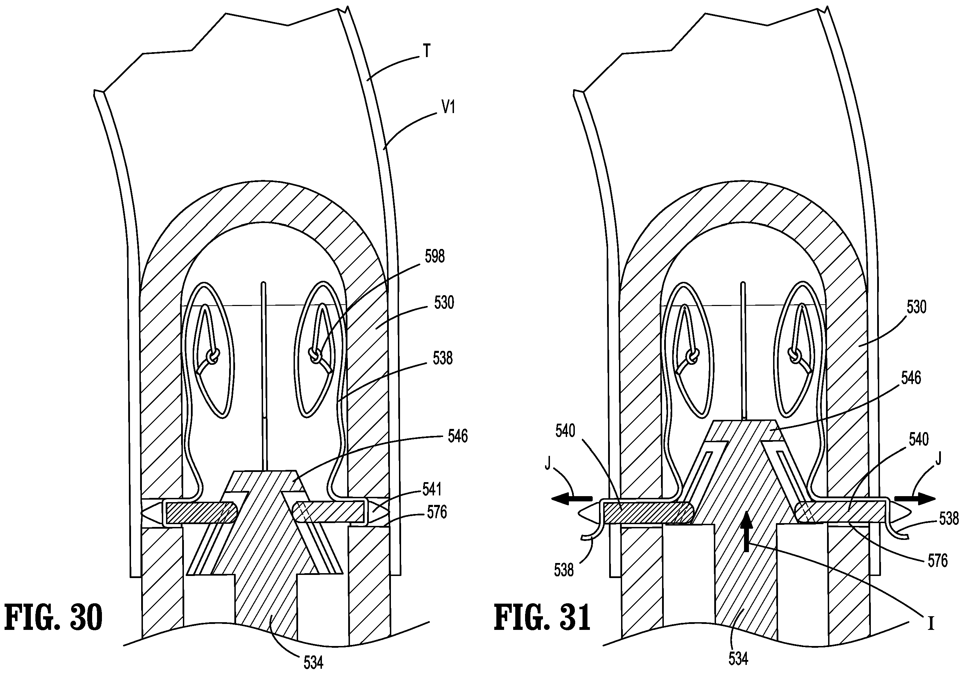

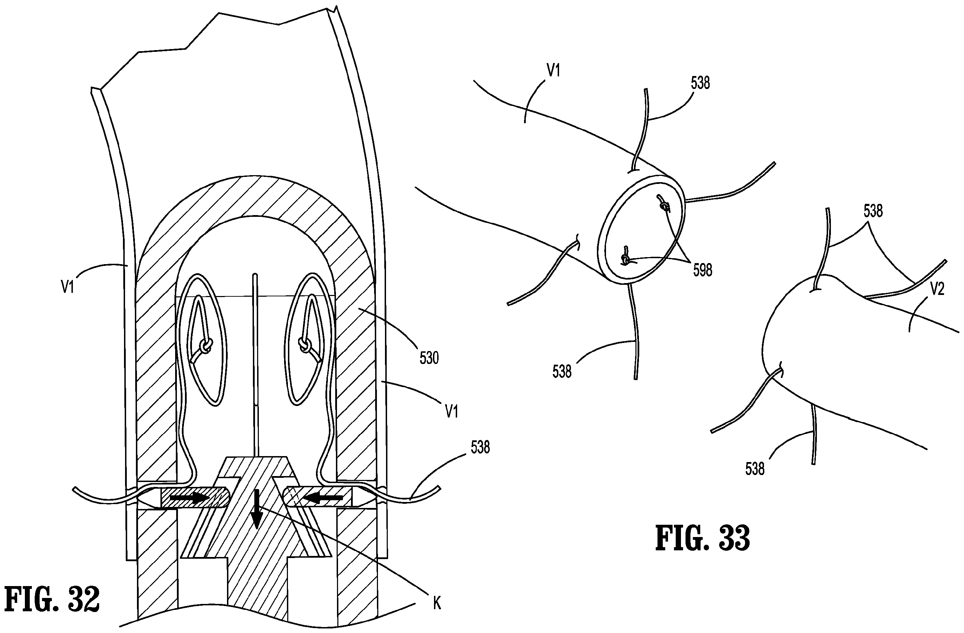

The drive members 534 (only one is shown) and the tissue piercing members 540 function in substantially the same manner as the drive members 134 (FIG. 15) and the tissue piercing members 140 described above regarding the suture deployment assemblies 102 and 104 (FIG. 6). More specifically, when the tissue piercing members 540 are in a non-deployed position, the tissue piercing members 540 are received in a small-diameter end of the cam member 546 (FIGS. 29 and 30). When the drive member 534 is moved in the direction of arrow "I" in FIG. 31 and the cam member 546 moves in relation to the tissue piercing members 540, the tissue piercing members 540 are driven outwardly in the direction of arrows "J" through the openings 576 in the capsules 530 (only one is shown) and through the tissue "T" of a first vessel portion "VI". After the tissue piercing members 540 advance the sutures 538 through the tissue "T", a clinician can separate the sutures 538 from the tissue piercing members 540 with, e.g., a grasper, and pull the sutures 538 further through the tissue. Once the sutures 538 are pulled further through the tissue "T", e.g., such that the knots 598 engage an inner surface of the tissue "T", the drive member 534 including the cam member 546 can be moved in the direction of arrow "K" in FIG. 32 to retract the tissue piercing members 540 in the direction of arrows "L". Receipt of the ribs 556 of the tissue piercing members 540 within the slots 554 (FIG. 29) in the side walls 552 defining longitudinal channels 550 within the cam member 546 of the drive member 534 forces the tissue piercing members 540 to withdraw from the tissue "T" and move back into the capsule 530 through the openings 576.

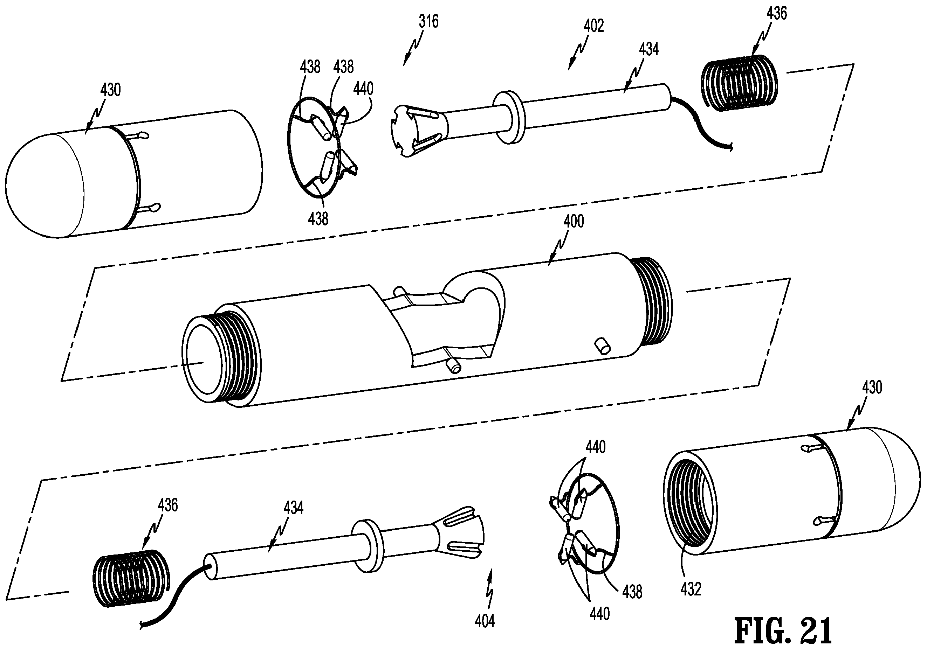

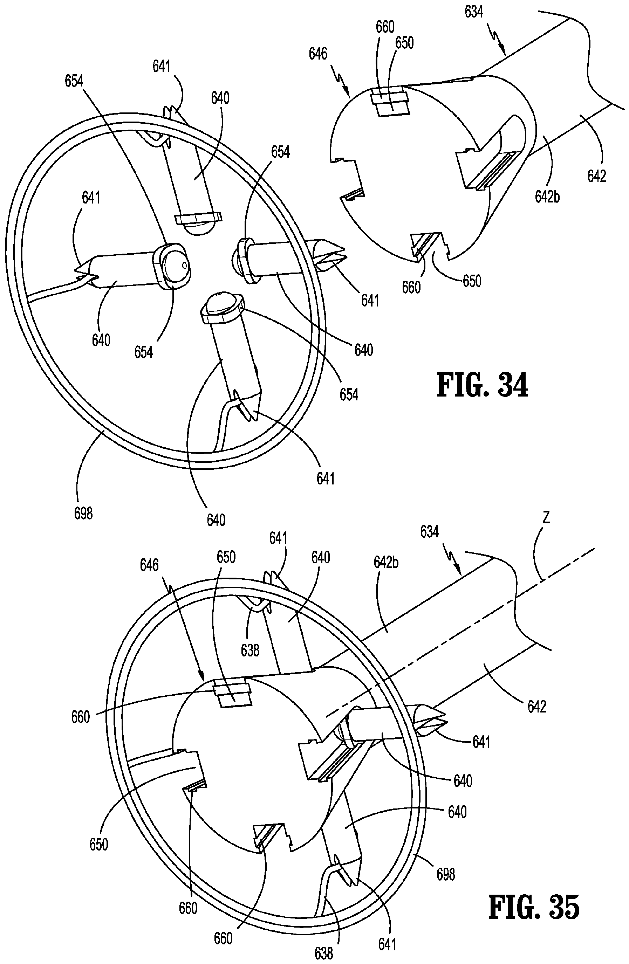

FIG. 33 illustrates the vessel portions "VI" and "V2" with the sutures 538 extending through the vessel portions "VI" and "V2". In this state, the ends of the sutures 138 positioned within the vessel portions "VI" and "V2" include knots 598 that prevent the sutures 538 from passing through openings in the vessel portions "VI" and "V2" formed by the tissue piercing members 540 (FIG. 32). The sutures 538 from the vessel portions "VI" and "V2" can be secured together to form the anastomosis. FIGS. 34 and 35 illustrate another version of the of a drive member and tissue piercing members of a suture deployment assembly of the anastomosis device 10 shown in FIG. 1. Each of the tissue piercing members is shown generally as 640 and includes a tapered tip 641 for piercing tissue. The drive members (only one is shown) shown generally as drive member 634 includes a cylindrical shaft 642 having a first end (not shown) and a second end 642b. The cylindrical shaft 642 defines a longitudinal axis "Z" (FIG. 35) and includes a cam member 646 supported on the second end 642b of the cylindrical shaft 642. The cam member 646 is substantially conical in shape and defines a plurality of longitudinally extending channels 650. Each of the longitudinally extending channels 650 receives the end of a respective one of the tissue piercing members 640 opposite to the tapered tip 641. The tissue piercing members 640 and the cam member 646 are substantially the same as the tissue piercing members 540 (FIG. 27) and cam member 546, respectively, except that the cam member 646 which is conical is secured to the drive member 634 with the large diameter portion of the cam member 634 spaced from the second end 642b of the cylindrical shaft 642 such as shown in regard to the drive member 434 in FIGS. 21-25. As such, these components will not be described in further detail herein.

Each of the tissue piercing members supports a suture 638. The sutures 638 are substantially similar to the sutures 538 (FIG. 26) except that the knot 598 (FIG. 33) is replaced by a suture loop 698 such as shown in regard to suture 438. More specifically, each of the sutures 638 is connected to each of the other sutures 638 by a suture loop 698. In some aspects of the disclosure, the suture loop 698 is received within an annular groove, e.g., annular groove 474 (FIG. 20) formed about the capsule (not shown) of the suture deployment assembly (not shown).

The tissue piercing members 640 include ribs 654 that are received within slots 660 formed in side walls 652 that define the longitudinally extending channels 650 of the cam member 646. As such, movement of the drive member 634 between retracted and advanced positions moves the tissue piercing members 640 between non-deployed and deployed positions. Except for the inclusion of the ribs 654 of the tissue piercing members 640 and the slots 660 defined in the cam member 646, the drive member 634 and the tissue piercing members 640 operate as described above regarding the drive members 434 and tissue piercing members 440 (FIG. 23). As such, operation of the drive member 634 and the tissue piercing members 640 are not described in further detail. Persons skilled in the art will understand that the devices and methods specifically described herein and illustrated in the accompanying drawings are non-limiting exemplary aspects of the disclosure. It is envisioned that the elements and features illustrated or described in connection with one exemplary aspect of the disclosure may be combined with the elements and features of another without departing from the scope of the disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

The invention may be described by reference to the following numbered paragraphs:-
1. An anastomosis device comprising:
   a handle assembly including an articulation trigger and a firing trigger;
   an elongate body defining a first longitudinal axis and having a proximal portion and a distal portion, the proximal portion coupled to the handle assembly; and
   an end effector supported on the distal portion of the elongate body, the end effector defining a second longitudinal axis and including:
      a housing pivotably supported on the distal portion of the elongate body, the housing having first and second end portions and movable from a stowed position in which the first and second longitudinal axes are aligned with each other to a deployed position in which the first and second longitudinal axes are substantially perpendicular to each other; and
      a first suture deployment assembly supported on the first end portion of the housing and a second suture deployment assembly supported on the second end portion of the housing, each of the first and second suture deployment assemblies including a capsule, a drive member, and a plurality of sutures, each of the plurality of sutures including a first end, a second end, and a tissue piercing member secured to the first end of each of the plurality of sutures, each of the capsules defining a first cavity that receives one of the drive members and the plurality of sutures, each of the capsules defining radial openings that communicate with the first cavity, wherein the drive member of each of the first and second deployment assemblies is engaged with the tissue piercing members and is movable from a first position to a second position to advance the tissue piercing members through the radial openings.
2. The anastomosis device of paragraph 1, wherein each of the drive members includes a first end and a second end, the second end of each of the drive members including a cam member that is engaged with the tissue piercing members such that movement of the drive members from their first positions to their second positions advances the tissue piercing members through the radial openings.
3. The anastomosis device of paragraph 2, wherein the cam member of each of the drive members is conically shaped.
4. The anastomosis device of paragraph 3, wherein each of the cam members defines channels, each of the channels receives one end of one of the tissue piercing members.
5. The anastomosis device of paragraph 1, wherein each of the first and second suture deployment assemblies includes a biasing member, the biasing members urging the drive members towards their first positions.
6. The anastomosis device of paragraph 1, wherein the elongate body includes an outer tube having a proximal portion and a distal portion, the distal portion of the outer tube including an inner surface that defines diametrically opposed longitudinal slots, and the housing includes a central portion including pivot members, the pivot members being received within the diametrically opposed longitudinal slots to facilitate pivotable movement of the end effector in relation to the outer tube between the stowed position and the deployed position.
7. The anastomosis device of paragraph 1, wherein the diametrically opposed longitudinal slots include closed distal ends.
8. The anastomosis device of paragraph 7, wherein the inner surface of the distal portion of the outer tube includes a transverse slot that communicates with each of the diametrically opposed longitudinal slots, and the housing includes a guide member that is received in each of the diametrically opposed longitudinal slots, the guide members movable through the transverse slots as the housing moves from the stowed position to the deployed position.
9. The anastomosis device of paragraph 4, wherein the channels define slots and the tissue piercing members include ribs that are received within the slots defined within the channels to retain the tissue piercing members within the slots defined within the channels.
10. The anastomosis device of paragraph 1, wherein movement of the drive member from second position back to the first position retracts the tissue piercing members to a position within the capsule.
11. The anastomosis device of paragraph 1, wherein the anastomosis device includes a firing rod including a proximal portion and a distal portion, the proximal portion of the firing rod coupled to the firing trigger and the distal portion of the firing rod engaged with the drive members of the first and second suture deployment assemblies, the firing rod movable in response to actuation of the firing trigger from a retracted position to an advanced position to move the drive members from their first positions to their second positions.
12. The anastomosis device of paragraph 2, wherein the first end of each of the drive members includes a tapered surface, the tapered surfaces of the drive members positioned adjacent to each other to define a wedge-shaped recess, the distal portion of the firing rod aligned with the wedge-shaped recess when the firing rod in in its retracted position.
13. The anastomosis device of paragraph 1, wherein the anastomosis device includes a firing cable including a proximal portion and a distal portion, the proximal portion of the firing cable coupled to the firing trigger and the distal portion of the firing cable engaged with the drive members of the first and second suture deployment assemblies.
14. The anastomosis device of paragraph 1, wherein the anastomosis device includes articulation links, each of the articulation links including a proximal portion coupled to the articulation trigger and a distal portion coupled to the housing, the articulation links movable from retracted positions to an advanced positions to move the housing from the stowed position to the deployed position.
15. An end effector comprising:
   a housing defining a longitudinal axis, the housing having first and second end portions and a central portion including diametrically opposed pivot members, the housing defining a first cavity; and
   a first suture deployment assembly supported on the first end portion of the housing and a second suture deployment assembly supported on the second end portion of the housing, each of the first and second suture deployment assemblies including a capsule, a drive member, and a plurality of sutures, each of the plurality of sutures including a first end, a second end, and a tissue piercing member secured to the first end, each of the capsules defining a second cavity that communicates with the first cavity of the housing, the drive member and the plurality of sutures received within the respective second cavities of the capsules of the first and second suture deployment assemblies, each of the capsules defining radial openings that communicate with the second cavities, wherein the drive member of each of the first and second deployment assemblies is engaged with the tissue piercing members and is movable from a first position to a second position to advance the tissue piercing member through the radial openings.
16. The end effector of paragraph 15, wherein each of the drive members includes a first end and a second end, the second end of each of the drive members including a cam member that is engaged with the tissue piercing members such that movement of the drive members from their first positions to their second positions advances the tissue piercing members through the radial openings.
17. The end effector of paragraph 16, wherein the cam member of each of the drive members is conically shaped and defines a plurality of channels, each of the plurality of channels receiving one end of one of the tissue piercing members.
18. The end effector of paragraphs 17, wherein each of the first and second suture deployment assemblies includes a biasing member, the biasing members urging the drive members to their first positions.
19. The end effector of paragraph 15, wherein the channels define slots and the tissue piercing members include ribs that are received within the slots defined within the channels to retain the tissue piercing members within the slots defined within the channels.
20. The end effector of paragraph 15, wherein movement of the drive member from second position back to the first position retracts the tissue piercing members to a position within the capsule.

## Claims

1. An anastomosis device comprising:
a handle assembly including an articulation trigger and a firing trigger;
an elongate body defining a first longitudinal axis and having a proximal portion and a distal portion, the proximal portion coupled to the handle assembly; and
an end effector supported on the distal portion of the elongate body, the end effector defining a second longitudinal axis and including:
a housing pivotably supported on the distal portion of the elongate body, the housing having first and second end portions and movable from a stowed position in which the first and second longitudinal axes are aligned with each other to a deployed position in which the first and second longitudinal axes are substantially perpendicular to each other; and
a first suture deployment assembly supported on the first end portion of the housing and a second suture deployment assembly supported on the second end portion of the housing, each of the first and second suture deployment assemblies including a capsule, a drive member, and a plurality of sutures, each of the plurality of sutures including a first end, a second end, and a tissue piercing member secured to the first end of each of the plurality of sutures, each of the capsules defining a first cavity that receives one of the drive members and the plurality of sutures, each of the capsules defining radial openings that communicate with the first cavity, wherein the drive member of each of the first and second deployment assemblies is engaged with the tissue piercing members and is movable from a first position to a second position to advance the tissue piercing members through the radial openings.

2. The anastomosis device of claim 1, wherein each of the drive members includes a first end and a second end, the second end of each of the drive members including a cam member that is engaged with the tissue piercing members such that movement of the drive members from their first positions to their second positions advances the tissue piercing members through the radial openings.

3. The anastomosis device of claim 2, wherein the cam member of each of the drive members is conically shaped.

4. The anastomosis device of claim 3, wherein each of the cam members defines channels, each of the channels receives one end of one of the tissue piercing members.

5. The anastomosis device of any preceding claim, wherein each of the first and second suture deployment assemblies includes a biasing member, the biasing members urging the drive members towards their first positions; and/or wherein the elongate body includes an outer tube having a proximal portion and a distal portion, the distal portion of the outer tube including an inner surface that defines diametrically opposed longitudinal slots, and the housing includes a central portion including pivot members, the pivot members being received within the diametrically opposed longitudinal slots to facilitate pivotable movement of the end effector in relation to the outer tube between the stowed position and the deployed position.

6. The anastomosis device of any preceding claim, wherein the diametrically opposed longitudinal slots include closed distal ends; preferably wherein the inner surface of the distal portion of the outer tube includes a transverse slot that communicates with each of the diametrically opposed longitudinal slots, and the housing includes a guide member that is received in each of the diametrically opposed longitudinal slots, the guide members movable through the transverse slots as the housing moves from the stowed position to the deployed position.

7. The anastomosis device of claim 4, wherein the channels define slots and the tissue piercing members include ribs that are received within the slots defined within the channels to retain the tissue piercing members within the slots defined within the channels.

8. The anastomosis device of any preceding claim, wherein movement of the drive member from second position back to the first position retracts the tissue piercing members to a position within the capsule; and/or wherein the anastomosis device includes a firing rod including a proximal portion and a distal portion, the proximal portion of the firing rod coupled to the firing trigger and the distal portion of the firing rod engaged with the drive members of the first and second suture deployment assemblies, the firing rod movable in response to actuation of the firing trigger from a retracted position to an advanced position to move the drive members from their first positions to their second positions.

9. The anastomosis device of claim 2, wherein the first end of each of the drive members includes a tapered surface, the tapered surfaces of the drive members positioned adjacent to each other to define a wedge-shaped recess, the distal portion of the firing rod aligned with the wedge-shaped recess when the firing rod in in its retracted position.

10. The anastomosis device of any preceding claim, wherein the anastomosis device includes a firing cable including a proximal portion and a distal portion, the proximal portion of the firing cable coupled to the firing trigger and the distal portion of the firing cable engaged with the drive members of the first and second suture deployment assemblies; and/or wherein the anastomosis device includes articulation links, each of the articulation links including a proximal portion coupled to the articulation trigger and a distal portion coupled to the housing, the articulation links movable from retracted positions to an advanced positions to move the housing from the stowed position to the deployed position.

11. An end effector comprising:
a housing defining a longitudinal axis, the housing having first and second end portions and a central portion including diametrically opposed pivot members, the housing defining a first cavity; and
a first suture deployment assembly supported on the first end portion of the housing and a second suture deployment assembly supported on the second end portion of the housing, each of the first and second suture deployment assemblies including a capsule, a drive member, and a plurality of sutures, each of the plurality of sutures including a first end, a second end, and a tissue piercing member secured to the first end, each of the capsules defining a second cavity that communicates with the first cavity of the housing, the drive member and the plurality of sutures received within the respective second cavities of the capsules of the first and second suture deployment assemblies, each of the capsules defining radial openings that communicate with the second cavities, wherein the drive member of each of the first and second deployment assemblies is engaged with the tissue piercing members and is movable from a first position to a second position to advance the tissue piercing member through the radial openings.

12. The end effector of claim 11, wherein each of the drive members includes a first end and a second end, the second end of each of the drive members including a cam member that is engaged with the tissue piercing members such that movement of the drive members from their first positions to their second positions advances the tissue piercing members through the radial openings.

13. The end effector of claim 12, wherein the cam member of each of the drive members is conically shaped and defines a plurality of channels, each of the plurality of channels receiving one end of one of the tissue piercing members; preferably wherein each of the first and second suture deployment assemblies includes a biasing member, the biasing members urging the drive members to their first positions.

14. The end effector of any of claims 11 to 13, wherein the channels define slots and the tissue piercing members include ribs that are received within the slots defined within the channels to retain the tissue piercing members within the slots defined within the channels.

15. The end effector of any of claims 11 to 14, wherein movement of the drive member from second position back to the first position retracts the tissue piercing members to a position within the capsule.
